(19) Europäisches Patentamt
European Patent Office
Office européen des brevets

(11) Publication number: **0 071 087**
**B1**

(12) **EUROPEAN PATENT SPECIFICATION**

(45) Date of publication of patent specification: **24.04.85**

(51) Int. Cl.$^4$: **C 12 Q 1/50**

(21) Application number: **82106303.9**

(22) Date of filing: **14.07.82**

(54) **Improved determination of creatine phosphokinase in body fluids.**

(30) Priority: **17.07.81 US 284269**

(43) Date of publication of application:
**09.02.83 Bulletin 83/06**

(45) Publication of the grant of the patent:
**24.04.85 Bulletin 85/17**

(84) Designated Contracting States:
**DE FR IT**

(56) References cited:
**EP-A-0 015 437**
**EP-A-0 022 432**
**EP-A-0 049 606**
**DE-A-1 930 059**
**US-A-4 012 286**
**US-A-4 042 462**
**US-A-4 220 714**

(73) Proprietor: **SERAGEN DIAGNOSTICS, INC.**
**P.O. Box 1210**
**Indianapolis Indiana 46206 (US)**

(72) Inventor: **Sanderson, James A.**
**5164 N. Illinois Street**
**Indianapolis Indiana 46208 (US)**

(74) Representative: **Patentanwälte Zellentin**
**Zweibrückenstrasse 15**
**D-8000 München 2 (DE)**

**EP 0 071 087 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Technical field

This invention relates to an improved method and composition for the determination of creatine phosphokinase.

Background art

The enzyme creatine phosphokinase, hereafter abbreviated CPK, is found in normal human serum and catalyzes the transfer of phosphate from creatine phosphate to adenosine diphosphate (ADP) to form adenosine triphosphate (ATP) and creatine. Analysis of CPK concentration in the serum is commonly used in the diagnosis of human diseases or disorders such as myocardial infarction, pulmonary infarction, cerebral infarction, hypothyroidism, liver disorder, skeletal muscle damage due to trauma or injury, and muscular dystrophy.

An assay method for CPK using the reverse action of creatine phosphokinase and adenylate kinase (AK) was developed by Oliver, Biochem. J. 61, 116 (1955). This method was later improved by Rosalki, J. Lab. Clin. Met. 61, 696 (1967), and Hess, et al., Am. J. Clin. Path 50, 89 (1968). Further modifications of the procedure permitted the use of nicotinamide adenine dinucleotide (NAD) instead of nicotinamide adenine dinucleotide phosphate (NADP) in the final ultraviolet detection step. See Okinaka, et. al., J. Lab and Clinical Med. 64, 299 (1964). The key reactions may be summarized as follows:

I. oxized inactive CPK+reduced thiol activator$\rightarrow$reduced active CPK+oxidized thiol activator

II. $\text{ADP+creatine phosphate} \xrightleftharpoons{\text{active CPK}} \text{ATP+ creatine}$

III. $\text{ATP+glucose} \xrightleftharpoons{\text{hexodinase}} \text{ADP+glucose-6-phosphate}$

IV. $\text{glucose-6-phosphate+NAD}^+ \xrightarrow{\text{glucose-6-phosphate dehydrogenase}} \text{6-phosphogluconate+NADH}$

To eliminate the need for ultraviolet detecting instrumentation the NADH formed in reaction IV above may be converted to $NAD^+$ with the subsequent reduction of the tetrazolium compound 2 - (p - iodophenyl) - 3 - (p - nitrophenyl) - 5 - phenyl tetrazolium chloride, herafter INT, to form the red formazan dye INTH via enzymatic catalysis by diaphorase. See Avigad and Levin, N, Europ. J. Biochem. 1, 102 (1969). This reaction may be expressed as follows:

V. $\text{NADH+INT} \xrightarrow{\text{diaphorase}} \text{INTH+NAD}^+$

Hereinafter the sequence of reactions I through V will be called the Oliver-Rosalki-formazan method. A body fluid, such as serum, plasma, lymphatic fluid or the like is analyzed for CPK activity using this method by adding test fluid to a prewarmed reconstituted lypholized substrate containing activator, primary substrates, intermediate substrates, final substrates, coupling enzymes, adenylate kinase inhibitor AMP, buffering compounds and stabilizers. The test fluid is incubated (at about 37°C) for a fixed period of time (usually about 10 minutes) and then the reaction is stopped by the addition of acid. Likewise, water is added to another substrate and run as a reagent blank. Control serum having a known CPK activity is used to determine the activity of the unknown test serum.

The Oliver-Rosalki-formazan method as currently in use has four major drawbacks. First, there is variable lag time in the activation of CPK by the thiol activator in reaction I described above. While this lag can be followed using sophisticated continuously monitoring colorimetric measuring equipment having a reagent blank vial and using a double beam, this lag is a disadvantage when an end point method is used as employed in batch analysis. Thus if less sophisticated colorimetric equipment is used for end point method analysis some variation in results is unavoidable in the method as currently employed. Second, there is a continuous increase in the reagent blank absorbance following reconstitution of the substrate because of the presence of all the components necessary for non-serum CPK chromogen production. Third, there is a difference between the reagent blank vial's absorbance and the test vial's absorbance due to the variable degree of solubilization of the final chromogen, INTH, in the presence of serum. This results in an inadequate reagent blank. Fourth, the control serum is unstable and displays variable activation times which necessitates labelling commercial serum containing CPK with wide ranges of activity.

U.S. Patent 4,012,286 describes a variation of the Oliver-Rosalki-formazan method which differs in three important respects. (1) The serum CPK is preactivated prior to the start of enzyme catalysis and chromogen production. (2) A surfactant has been added to the enzyme substrate to aid in the solubilization of the chromogen eliminating the positive solubilization of serum on INTH. (3) As a result of the improved

chromogen solubilization in the absence of serum the control serum has been replaced by an intermediate reaction product, i.e., a pure chemical standard, which is subsequently converted to the final chromogen.

In a general CPK assay as recommended by F. Meiattini, G. Giannini and P. Tarli [Clin. Chem. *24*, 498 (1978), U.S. Patent 4,220,714] 2 m mole/liter AMP, 2m mole/liter ADP and 6m mole/liter sodium fluoride are used for optimum effect; however, residual AK activity is pronounced. Meiattini et. al., disclose an AMP/ADP ratio of from 1 to about 10 however, a ratio of 1 was found to produce maximum inhibition of the interfering AK enzyme.

It has been found, contrary to Meiattini, that a ratio of AMP/ADP exceeding 10, and preferably 11—60, (most preferably 15—25), in the presence of fluoride ion, results in a superior composition for the inhibition of AK when employed in a diagnostic assay system for CPK-MB isoenzyme, utilizing the inhibiting antibody approach as described by Neumeir et. al., Clin. Chem., Acta *73*, 445 (1976) and Gerhardt et. al., Clin. Chem. Acta *78*, 29 (1977), and either the traditional Oliver-Rosalki-formazan assay reaction sequence or the tetrazolium coupled improvement of U.S. Patent 4,012,286. This improved inhibition eliminates the need for serum blanks as required by the prior art methodology with only a small compromising reduction in CPK-B turnover rate.

Disclosure of invention

In the Neumeir and Gerhart methodology, the residual "B" subunit activity of the "MB" CPK isoenzyme activity of serum is measured via a modified Oliver-Rosalki coupled ultraviolet (UV) spectrophotemetric assay following treatment of the serum samples with inhibiting antibodies to the M subunit of the predominate serum CPK isoenzyme dimers MM and MB.

Immuno inhibition is performed in a reaction mix containing all of the CPK activity assay components, except the primary substrate creatine phosphate (CP). During this incubation phase, not only is complexation of M antibody with M-CK subunits occurring, but serum adenylate kinase, activated by sulfhydryl reductants in the CPK assay mix, is catalyzing the interfering reaction of disproportionating of adenosine diphosphate (ADP), the other primary CPK substrate present in the mixture, to form adenosine monophosphate (AMP) and adenosine triphosphate (ATP). ATP, the same product of CPK catalysis, is subsequently coupled to the end product production of reduced nicotinamide adenine dinucleotide (NADH).

AK interference is minimized with the use of the transition state complex-like compound diadenosine pentaphosphate (DAPP). However, insufficient inhibition of AK, found in high concentrations in erythrocytes and hence found in commonly observed hemolyzed serum samples, has mandated that serum blanks (i.e. the assay minus CP) be monitored after immuno-inhibition has occurred. Subsquently CPK-B catalysis is monitored following CP addition.

Formerly, AK interference was not a problem in CPK isoenzyme determinations involving serum component separation prior to assay, of the classical electrophoretic and ion-exchange methods. AK was physically separated from the isoenzyme of interest prior to activity staining or eluate analysis. With the use of an inhibiting antibody, the time-consuming separation of CPK isoenzymes is avoided but the need for improved AK interference is critical, especially for applicability to many automated clinical instruments not designed for serum blank analyses.

The improvement described herein involves utilization of an AMP/ADP ratio, drastically different from that used in conventional CPK assays, with the optional additional inclusion of fluoride to impart in total:

(1) dramatically improved inhibition of the adenylate kinase interference

(2) a small compromising decrease in measured CPK-B activity due to the enhanced competitive inhibition by AMP at a lower level of ADP.

Table I illustrates the utilization of a high AMP/ADP ratio in inhibiting purified human erythrocyte adenylate kinase. Residual AK activity is measured in reagent systems containing all necessary components of the Oliver-Rosalki CPK assay. The systems vary as a function of three components: AMP, ADP, and sodium fluoride. The data points cover a 1.43—5.72m Mole/liter ADP range and a 2.9—29.2m Mole/liter AMP range plus or minus 6m Mole/liter NaF. As can be seen in Table I, the over-riding parameter that dictates extent of inhibition of AK, or conversely the residual AK activity, is the AMP/ADP ratio.

Best mode for carrying out the invention

Pre-activation of the inactive CPK is accomplied by a reduced thiol activator such as for example, glutathione, dithiothreitol, N-acetyl cysteine, or cysteine (Reaction I above) prior to the beginning of enzyme catalysis and chromogen production. Sufficient thiol activator must be present to activate all of the CPK present in the body fluid. Therefore an excess of thiol activator is generally employed to assure the total activation of all CPK present. The CPK-activating amount of the thiol activator may be determined by the simple expedient of measuring the intensity of color produced with varying amounts of added activator when samples having known concentrations of CPK are employed. Preactivation eliminates the inacurracy resulting from the variable activation time of serum CPK because the CPK is fully active before coming into contact with the primary substrate ADP. This has been found to linearize chromogen production once the enzymatic reaction begins.

Side reactions which result in non-serum CPK chromogen production are also minimized by pre-activation of the CPK in the absence of the final intermediate substrates INT and ADP. Three

chromogen contributing side reactions which can lead to inaccurate results are (a) the reduction of INT by the thiol activator rather than by NADH, (b) the catalysis of ADP by CPK present as a contaminant in the coupling enzymes glucose-6-phosphate dehydrogenase and hexokinase, and (c) the fact that the inhibitor AMP of adenylate kinase does not completely eliminate adenylate kinase interference found in the coupling enzymes of (b) above. By eliminating both ADP and INT from the pre-activation mixture these side reactions are unable to take place prior to the beginning of the CPK enzymatic reaction.

The improved Oliver-Rosalki-formazan method therefore incorporates a unique sequence of steps in the carrying out of this method. In summary these steps are:

1. Preactivation step—The sample is mixed with a partial substrate containing a thiol activator, preferably glutathione, primary substrates, intermediate substrates, coupling enzymes, buffering compounds and stabilizers. However, missing from the partial substrate are the primary substrate ADP and the final substrate INT. The inhibitors AMP and a fluoride such as a alkali metal fluoride, for example, potassium fluoride or preferably sodium fluoride, may also, be added later, however, preferably it is added in the pre-activation step. By utilizing from 5m mole-liter to 8m mole-liter fluoride ion, preferably about 6m mole/liter, noncompetitive AK inhibition is acheived without precipitating magnesium ion, a necessary cofactor in the Oliver-Rosalki results assay. The pre-activation mixture is incubated, typically in a water bath or heating block at a temperature of from about 25° to about 40°C, 30° to 37°C being preferred, for a length of time sufficient to optimally activate the CPK, generally about 3 to 5 minutes. In the preparation of the materials for the preactivation step it is preferred to employ two separation compositions, a first lypholyzed partial substrate composition containing activator, creatine phosphate NAD, glucose, hexokinase, glucose-6-phosphate dehydrogenase, and diaphorase and a second reconstituting reagent containing a fluoride, a buffer with a pH of between 6 and 7 and surfactant. In addition, when it is desired to eliminate the contribution of the "M" subunit of the CPK isoenzyme dimers "MM" and "MB" from the assay, the lypholized partial substrate can contain anti CPK-M inhibiting antibodies. The elimination of the "M" subunit contribution in the CPK assay is particularly valuable in the diagnosis of myocardial infarction. When the ingredients for the partial substrate are thus prepared as two separate compositions, both such compositions are stable for long periods of time prior to use. The separate compositions are mixed to provide the reconstituted partial substrate used in the pre-activation step.

2. Enzyme catalyis step—A starting reagent, containing the primary substrate ADP, a tetrazolium salt dye such as INT, and the inhibitor AMP is mixed with the incubated pre-activation mixture.

3. Addition of stop reagent—a major advantage of the improved method is that it may be used to accurately measure CPK activity on clinical grade colorimeters. Usually this will require that the chromogen producing reaction be stopped by the addition of acid, as for instance hydrochloric acid (HCl), after a time sufficient to allow adequate chromogen development. Twenty five minutes has been found to give satisfactory chromogen development.

The above reactions take place generally at a pH of between 6 and 7 with a pH of from 6.3 to 6.5 being preferred.

The body fluids which can be assayed by the improved CPK assay procedure of this invention include blood serum, blood plasma, cerebrospinal fluid (CSF), lymphatic fluid and tissue homogenates. The various body fluids to be assayed by the methodology of this invention are to be from a human source although the general CPK assay is useful whenever animal body fluids are to be assayed. The preferred body fluid is human blood serum.

Preferred compositions of the above are as follows:

Example 1
Preactivation reagent (reconstituted*)

| | |
|---|---|
| Glutathione activator | 5 m mole/liter |
| Creatine phosphate (from yeast) | 34.5 m mole/liter |
| Glucose | 12 m mole/liter |
| Hexokinase (from yeast) | 5.5 U/test |
| Glucose-6-phosphate dehydrogenase (from Leuconostoc mesenteroides) | 2.24 U/test |
| Diaphorase (from Clostriduim kluyveri) | 3 U/test |
| Magnesium acetate | 14.4 m mole/liter |
| 3-(N-morpholino)propane sulfonic acid buffer | 82.18 m mole/liter |
| Surfactant (Emulphor EL 620) | 0.025% W/V |
| Nicotinamide adenine dinucleotide (from yeast) | 2.77 m mole/liter |
| Anti CPK-M inhibiting antibody (from goat serum) | ≤2000 U/liter |
| NaF | 6 m mole/liter |

*The preparation above represents the composition in the reconstituted state. In a preferred embodiment of the lypholyzed composition, the sulfonic acid buffer, surfactant and sodium fluoride, would be absent and added later during reconstitution.

Example 2
Starting reagent

| | |
|---|---|
| Adenosine-5′-diphosphate (from yeast) | 8.6 m mole/liter |
| Adenosine-5′-monophosphate (from yeast) | 175 m mole/liter |
| 2-(p-iodophenyl)-3-(p-nitrophenyl)-5-phenyltetrazoluim chloride | 4.91 m mole/liter |

Example 3

A preferred embodiment of the improved Oliver-Rosalki-formazan method is carried out as follows:

a) Transfer 1.0 ml. of the preactivation reagent of Example 1 into each of 3 empty vials appropriately marked patient, blank and standard.

b) Incubate the vials for approximately 10 minutes at 37°C.

c) Mix 0.10 ml of the patients serum into the patients' vial and 0.10 ml of distilled water into the blank vial.

d) Incubate all three vials with about 0.66 ml of the starting reagent (Example 2 above) in a separate container in a 37°C heat source for about 3—5 minutes.

e) Mix 0.2 ml of the pre-warmed Starting Reagent to all three vials and continue incubation.

f) Add 0.10 ml of the chemical standard (glucose-6-phosphate 2 m mole/liter) to the standard vial.

g) Twenty five minutes after the addition of the starting reagent add 4.0 ml of the stopping reagent (hydrochloric acid 40 m mole/liter containing 0.6% W/V Emulphor EL 620) to each vial.

h) Using a colorimeter or spectrophotometer set at 500 nm run an analysis of the material. The indicating device on the colorimeter should have been previously set to read zero absorbance for distilled water. The absorbance of each of the three vials is read on the colorimeter and recorded.

The preferred way of expressing enzyme concentration is in International Units/liter (U/liter). One International Unit is the amount of enzyme that will transform one micromole of substrate per minute under standard conditions. Therefore the concentration of CPK in the patient's sample may be calculated using the following formula:

$$U/liter=\frac{A\ (Patient's\ vial)-A\ (Blank\ vial)}{A\ (Standard\ vial)-A\ (Blank\ vial)}\times Conc.\ of\ standard\ in\ U/liter$$

wherein A=absorbance.

A chemical standard using glucose-6-phosphate in a concentration of 2 m moles/liter which is equivalent to 80 U/liter of serum has been found satisfactory as a standard in the above procedure. Therefore in the above formula the value U/liter would be used as the concentration of standard in U/liter.

Example 4

The data of Table 1 demonstrate the effects of varying amounts of AMP and ADP and the presence or absence of 6 m mole/liter sodium fluoride on the assay of CPK. The reagents of Example 1 and 2 were utilized with the AMP and ADP concentration listed in the table, with or without 6 m mole/liter sodium fluoride.

TABLE 1

Inhibition of purified human erythrocyte adenylate kinase in a CPK-UV assay as a function of (AMP), (ADP), and (NaF)

| (ADP) (mM) | (AMP) (mM) | AMP/ADP | Measured residual adenylate kinase (U/l at 37°C) (−NaF) | (+NaF) |
|---|---|---|---|---|
| 1.43 | 0 | — | 354 | 327 |
| 1.43 | 2.9 | 2.0 | 103 | 24 |
| 1.43 | 5.8 | 4.1 | 61 | 13 |
| 1.43 | 7.3 | 5.1 | 56 | 10 |
| 1.43 | 10.2 | 7.1 | 35 | 7.7 |
| 1.43 | 14.6 | 10.2 | 333 | 5.3 |
| 1.43 | 21.9 | 15.3 | 19 | 4.6 |
| 1.43 | 29.2 | 20.4 | 17 | 1.7 |
| 2.86 | 0 | — | 394 | 398 |
| 2.86 | 2.9 | 1.0 | 166 | 51 |
| 2.86 | 5.8 | 2.0 | 126 | 26 |
| 2.86 | 7.3 | 2.6 | 93 | 22 |
| 2.86 | 10.2 | 3.6 | 84 | 17 |
| 2.86 | 14.6 | 5.1 | 64 | 13 |
| 2.86 | 21.9 | 7.7 | 32 | 10 |
| 2.86 | 29.2 | 10.2 | 28 | 5.3 |
| 5.72 | 0 | — | 459 | 460 |
| 5.72 | 2.9 | 0.51 | 203 | 108 |
| 5.72 | 5.8 | 1.0 | 186 | 55 |
| 5.72 | 7.3 | 1.3 | 126 | 54 |
| 5.72 | 10.2 | 1.8 | 114 | 37 |
| 5.72 | 14.6 | 2.6 | 108 | 31 |
| 5.72 | 21.9 | 3.8 | 66 | 21 |
| 5.72 | 29.2 | 5.1 | 62 | 13 |

Example 5

Table II illustrates the serum blank minimization with the improved CPK-B UV assay composition. Human serum samples, many being hemolytic due to sampling from patients undergoing heart surgery, were obtained from a local coronary care unit. Samples, stored refrigerated following collection, were assayed within three days using a UV test patterned after the Neumier et al. and Gerhardt et al. approached (prior art) differing essentially from the instant assay composition in that AMP/ADP ratio is 2.5 and in the absence of fluoride ion. Both the "prior art" composition and the assay composition of this invention containing the superior AK inhibition composition (Example 1 and 2) were prepared lacking creatine phosphate.

In the prior art assay system as expected, hemolytic sera exhibited greater sera blanks than nonhemolytic sera owing to erythrocyte AK. However, with the superior AK inhibition composition of the

present system, little if any serum blank is evident. With values of "B" activity >8 U/l @ 37°C suggesting myocardial damage, the prior art serum blanks are significantly large to mandate routine determination, whereas their measurement is unnecessary for the present system.

TABLE II
Serum blank determinations as a function of UV CPK-B reagent

| Sample #[1] | Measured serum blank (U/l at 37°C) Prior art[2] | Improved assay[3] |
|---|---|---|
| 1[4] | 7.0 | 0.6 |
| 2 | 3.4 | 0.1 |
| 3 | 5.8 | 0.6 |
| 4 | 5.5 | 0.4 |
| 5 | 4.6 | 0.4 |
| 6 | 2.9 | 0.4 |
| 7 | 6.2 | 0.3 |
| 8 | 2.8 | 0.4 |
| 9 | 6.5 | 0.7 |
| 10 | 3.6 | 1.0 |
| 11 | 6.5 | 0.5 |
| 12 | 3.9 | 0.6 |
| 13 | 3.6 | 0.1 |
| 14 | 3.4 | 0.1 |
| 15 | 7.2 | 0.3 |
| 16 | 3.7 | 0 |
| 17 | 4.0 | 0.2 |
| 18 | 4.6 | −0.7 |
| 19 | 3.6 | −0.1 |
| 20 | 6.2 | 0.3 |
| 21 | 3.0 | 0.3 |
| 22 | 41.6 | 1.2 |
| 23 | 5.2 | 0.4 |
| 24 | 1.9 | 0.6 |
| 25 | 3.7 | 0.5 |
| 26[4] | 5.4 | −0.2 |
| 27[4] | 7.9 | 0.3 |
| 28[4] | 8.5 | 0.3 |

TABLE II (cont'd.)

| Sample #[1] | Measured serum blank (U/l at 37°C) Prior art[2] | Improved assay[3] |
|---|---|---|
| 29[4] | 9.7 | −0.7 |
| 30[4] | 5.8 | 0.7 |
| 31[4] | 18.2 | 0.7 |
| 32 | 3.4 | 0.5 |
| 33 | 5.4 | 0.6 |
| 34 | 4.5 | 0.8 |
| 35 | 5.4 | 0 |
| 36 | 3.4 | 0.2 |
| 37 | 3.0 | 0.2 |
| 38 | 4.7 | 0.5 |
| 39 | 4.1 | 0.5 |
| 40 | 4.1 | 0.5 |
| 41 | 4.8 | 0.3 |
| 42 | 4.1 | 0.4 |
| 43 | 3.7 | 0.6 |
| 44 | 5.0 | 0.4 |
| 45 | 4.0 | 0.4 |
| 46 | 3.5 | 0.8 |
| 47 | 4.1 | 0.7 |
| 48 | 4.2 | 0.9 |
| 49 | 7.7 | 0.4 |
| 50 | 3.7 | 0.2 |
| 51 | 3.9 | 0.4 |
| 52 | 4.9 | 0.5 |
| 53 | 2.8 | 0.3 |

[1]Serum samples from a coronary care unit.
[2]Prior art assay system minus creatine phosphate.
[3]Present assay system minus creatine phosphate (Example 3).
[4]Hemolyzed sample.

## Claims

1. A method for determining creatine kinase activity in a body fluid wherein said determination is made utilizing the adenosine-5'-diphosphate mediated conversion of creatine phosphate to creatine, characterized in the utilization of an adenosine - 5' - monophosphate/adenosine - 5' - diphosphate ratio of from 15 to 25.

2. The method of claim 1 wherein said adenosine-5'-monophosphate/adenosine-5'-diphosphate ratio is about 20.

3. The method of claim 1 wherein said body fluid is human blood serum.

4. The method of claim 1 wherein fluoride ion is present.

5. A kit for the determination of creatine phosphokinase comprising a first pre-activation composition having a thiol activator, creatine phosphate hexokinase, glucose-6-phosphate dehydrogenase, glucose, nicotamide adenine dinucleolide, diaphorase, an fluoride ion and a second composition having a tetrazolium salt dye and adenosine - 5' - monophosphate and adenosine - 5' - diphosphate in a ratio of from 15 to 25.

6. A kit of claim 5 wherein the adenosine-5'-monophosphate/adenosine-5'-diphosphate ratio is about 20.

**Patentansprüche**

1. Ein Verfahren zur Bestimmung der Kreatinkinase-Aktivität in einer Körperflüssigkeit, bei dem die genannte Bestimmung unter Ausnützung der durch adenosin - 5' - diphosphat vermittelten Umwandlung von Kreatinphosphat in Kreatin erfolgt, gekennzeichnet durch die Anwendung eines Adenosin - 5' - monophosphat/Adenosin - 5' - diphosphat - Verhältnisses von 15 bis 25.

2. Verfahren nach Anspruch 1, bei dem das genannte Adenosin - 5' - monophosphat/Adenosin - 5' - diphosphat - Verhältnis etwa 20 beträgt.

3. Verfahren nach Anspruch 1, bei dem die genannte Körperflüssigkeit ein menschliches Blutserum ist.

4. Verfahren nach Anspruch 1, bei dem Fluoridionen anwesend sind.

5. Ein Kit zur Bestimmung von Kreatinphosphikinase, der eine erste Voraktivierungs-Zusammensetzung mit einem Thiol-Aktivator, Kreatinphosphat, Hexokinase, Glucose - 6 - phosphatdehydrogenase, Glucose, Nicotinamid - adenin - dinucleotid, Diaphorase und Fluoridionen sowie eine zweite Zusammensetzung mit einem Tetrazolium-salz-Farbstoff und Adenosin - 5' - monophosphat udn Adenosin - 5' - diphosphat in einem Verhältnis von 15 bis 25 enthält.

6. Kit nach Anspruch 5, bei dem das Adenosin-5'-monophosphat/Adenosin - 5' - diphosphat - Verhältnis etwa 20 beträgt.

**Revendications**

1. Procédé de détermination de l'activité de créatine-kinase dans un fluide physiologique, dans lequel on effectue ladite détermination en utilisant la conversion de phosphate de créatine en créatine avec l'assistance de 5' - diphosphate d'adénosine, caractérisé en ce qu'on utilise un rapport de 5' - monophosphate d'adénosine au 5' - diphosphate d'adénosine compris entre 15 et 25.

2. Procédé selon la revendication 1, dans lequel ledit rapport 5' - monophosphate d'adénosine/5' - diphosphate d'adénosine est d'environ 20.

3. Procédé selon la revendication 1, dans lequel ledit fluide physiologique est le sérum de sang humain.

4. Procédé selon la revendication 1, dans lequel est présent l'ion fluorure.

5. Kit pour la détermination de créatine-phosphokinase, comprenant une première composition de préactivation comportant un thiol activant, un phosphate de créatine, l'hexokinase, la 6 - phosphate de glucose déshydrogénase, le glucose, le nicotinamide - adénine - dinucléotide, la diaphorase et un ion fluorure et une seconde composition comportant un colorant de sel de tétrazolium et le 5' - monophosphate d'adénosine et 5' - diphosphate d'adénosine dans un rapport de 15 à 25.

6. Kit selon la revendication 5, dans laquelle le rapport 5'-monophosphat d'adénosine/5'-diphosphate d'adénosine est d'environ 20.